(19) Europäisches Patentamt
European Patent Office
Office européen des brevets

(11) Numéro de publication: **0 007 486**
**B1**

(12) **FASCICULE DE BREVET EUROPEEN**

(45) Date de publication du fascicule de brevet: **26.08.81**

(21) Numéro de dépôt: **79102278.3**

(22) Date de dépôt: **04.07.79**

(51) Int. Cl.³: **C 07 C 35/37,**
A 23 L 1/226, A 23 L 2/26,
A 24 B 15/30, A 61 K 7/46,
C 07 C 69/06, C 07 C 69/14

(54) **Composés oxygénés dérivant du tricyclo(6.2.1.0)undécane, leur utilisation à titre d'ingrédients parfumants respectivement aromatisants, et compositions contenant lesdits composés.**

(30) Priorité: **05.07.78 CH 7320/78**

(43) Date de publication de la demande:
**06.02.80 Bulletin 80/3**

(45) Mention de la délivrance du brevet:
**26.08.81 Bulletin 81/34**

(84) Etats Contractants Désignés:
**CH DE FR GB IT NL SE**

(56) Documents cités:
**DE - A - 2 362 877**
**DE - A1 - 2 737 525**
**FR - A - 1 414 969**
**FR - A1 - 2 236 828**

(73) Titulaire: **FIRMENICH SA**
**Case postale 239**
**CH-1211 Genève 8 (CH)**

(72) Inventeur: **Skorianetz, Werner**
**Rte du Mandement**
**CH-1249 Dardagny/Ge (CH)**
Inventeur: **Ohloff, Günther**
**Ch. de la Chapelle 13**
**CH-1233 Bernex/Ge (CH)**

(74) Mandataire: **Schmied-Kowarzik, Volker, Dr. et al,**
**Patentanwälte P. Wirth V. Schmied-Kowarzik G. Dannenberg P. Weinhold D. Gudel S. Schubert**
**Siegfriedstrasse 8**
**D-8000 München 40 (DE)**

**EP 0 007 486 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Composés oxygénés dérivant du tricyclo[6.2.1.$0^{2,7}$]undécane, leur utilisation à titre d'ingrédients parfumants, respectivement aromatisants, et compositions contenant lesdits composés

L'invention se rapporte à des composés oxygénés nouveaux, dérivant du tricyclo[6.2.1.$0^{2,7}$]-undécane utiles à l'industrie des parfums et arômes, notamment à titre d'ingrédients parfumants ou aromatisants. Elle a notamment pour objet des composés de formule

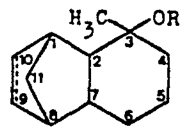

(I)

possédant une simple ou double liaison dans la position indiquée par les pointillés et dans laquelle le symbole R représente un atome d'hydrogène ou un radical acyle dérivant d'un acide carboxylique saturé ou insaturé contenant de 1 à 6 atomes de carbone.

L'invention a également pour objet une composition parfumante ou aromatisante contenant à titre d'ingrédient actif un composé de formule (I) telle que définie ci-dessus.

L'invention se rapporte en outre à l'utilisation desdits composés de formule (I) à titre d'ingrédients parfumants, respectivement aromatisants, pour la préparation de parfums, de produits parfumés, d'arômes artificiels ou pour l'aromatisation d'aliments, de boissons, de préparations pharmaceutiques et du tabac.

Il est de tradition, dans la parfumerie, d'utiliser des produits d'origine naturelle pour l'élaboration de compositions parfumantes. On a notamment recours à l'emploi d'extraits d'origine végétale ou animale tels que les baumes, les absolus, les concrètes ou les huiles essentielles par exemple. Etant donné l'évolution que connaît actuellement l'industrie des parfums, évolution liée entre autres à un emploi toujours plus étendu de produits cosmétiques et au parfumage de produits techniques tous les jours plus nombreux, cette dernière est fort souvent confrontée au problème de la raréfaction, voire de la disparition momentanée de certains produits d'origine naturelle, les huiles essentielles par exemple. Cela se traduit généralement par des ruptures de stock et une augmentation du prix de tels produits, conséquences fort dommageables tant pour l'industrie que pour le consommateur.

Illustrant ce type de situation, on peut citer par exemple l'huile essentielle de sauge sclarée (Salvia Sclarea) ou l'huile essentielle de marjolaine douce (Origanum Majorana) dont l'emploi est fort recherché dans le domaine de la parfumerie dite masculine. Ces huiles essentielles, dont la production, saisonnière, dépend fortement des conditions climatiques, sont au demeurant fort chères ce qui fait que l'on ne peut envisager leur emploi d'une façon aussi étendue que le permettrait le marché. Il est donc capital de pouvoir recourir à l'emploi de produits de remplacement, c'est-à-dire, de corps chimiques synthétiques odorants permettant de reproduire tout ou partie de l'effet olfactif développé par ces huiles essentielles.

De tels corps chimiques ont en effet l'avantage de pouvoir être préparés en quantités quasi illimitées, tout en présentant une qualité olfactive parfaitement reproductible alors que celle d'une huile essentielle peut fortement dépendre des conditions d'extraction ou de purification par exemple.

L'invention a précisément pour objet de proposer à l'homme de l'art une série de corps chimiques odorants possédant d'intéressantes propriétés organoleptiques et permettant notamment, selon les cas, de reproduire de façon satisfaisante certains des effets olfactifs d'une huile essentielle de sauge sclarée ou de marjolaine douce par exemple.

Ce fait est d'autant plus étonnant que rien dans l'état de la technique antérieure ne permettait de supposer que des composés chimiques possédant la structure ci-dessus présenteraient une note olfactive telle que décrite plus haut. Le composé de formule

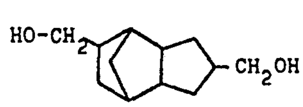

est en effet décrit dans l'art comme possédant une odeur musquée (voir à ce sujet DE—OS no. 23 07 627). Les composés de formules

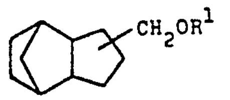

et

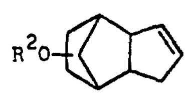

($R^1$ = H, alkyle ou acyle) ($R^2$ = alkyle ou alcényle)

se définissent par une odeur de type fleuri, fruité et boisé, respectivement de type fruité, vert et balsamique (voir à ce sujet DE—OS no. 26 54 268, respectivement DE—OS no. 26 42 519).

Le composé de formule

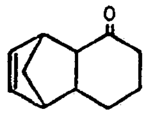

finalement, est décrit comme ayant une odeur de type aromatique, médicinal, rappelant celle de l'absinthe ou du liatris (voir à ce sujet DE—OS no. 27 37 525).

Dans la formule (I) telle que définie plus haut, le symbole R peut notamment représenter un radical formyle, acétyle, propionyle, butyryle, isobutyryle ou acrylyle par exemple, ou un atome d'hydrogène. A titre d'exemples de composés de formule (I) présentant un intérêt tout particulier on peut citer l'acétate de 3-méthyl-tricyclo[6.2.1.$0^{2,7}$]undéc-9-ène-3-yle, l'acétate de 3-méthyl-tricyclo-[6.2.1.$0^{2,7}$]undéc-3-yle et le formiate de 3-méthyl-tricyclo[6.2.1.$0^{2,7}$]undéc-9-ène-3-yle.

L'acétate de 3-méthyl-tricyclo[6.2.1.$0^{2,7}$]undéc-9-ène-3-yle plus particulièrement, de formule

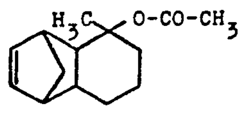

développe une odeur originale, tout à la fois fraîche et verte, caractéristique de certains des effets olfactifs de l'huile essentielle de sauge sclarée. Ledit composé est de ce fait très apprécié pour la préparation de compositions parfumantes variées, notamment de type vert, frais, chypré ou boisé par exemple. En parfumerie dite masculine, son addition à une base parfumante par exemple confère une note originale et montante très caractéristique.

Ces qualités olfactives se retrouvent de façon quasi identique au niveau du dérivé saturé correspondant, l'acétate de 3-méthyl-tricyclo[6.2.1.$0^{2,7}$]undéc-3-yle. Le formiate de 3-méthyl-tricyclo-[6.2.1.$0^{2,7}$]undéc-9-ène-3-yle s'enrichit en outre d'une note de type boisé, ambré et aromatique et développe des effets olfactifs se rapprochant de ceux de l'huile essentielle de marjolaine douce.

Les composés de formule (I) peuvent en fait s'utiliser aussi bien en parfumerie fine que pour la préparation de produits parfumés tels que savons, détergents, produits d'entretien ou produits cosmétiques par exemple. Lors de la préparation de compositions parfumantes, les effets les plus intéressants s'obtiennent en utilisant lesdits composés à raison de 0,5 à 20% environ du poids de ladite composition. Il demeure entendu cependant que des quantités supérieures ou inférieures aux limites données ci-dessus peuvent être également envisagées, notamment lors de la préparation de coeurs ou de bases parfumantes.

Dans le domaine des arômes, certains des composés de formule (I) développent une note gustatvie tout à la fois verte, boisée et terreuse de type pin, rappelant notamment celle de l'acétate de bornyle, du vétiver ou du cèdre. Ils peuvent être de ce fait utilisés pour la préparation d'arômes artificiels variés et pour l'aromatisation d'aliments, de boissons, de préparations pharmaceutiques et du tabac. Lors de la préparation d'arômes artificiels, lesdits composés peuvent s'utiliser à raison d'environ 0,5 à 5% par rapport au poids de l'arôme considéré.

Les composés de formule (I) peuvent être aisément préparés à partir de tricyclo[6.2.1.$0^{2,7}$]undéc-9-ène-3-one, après réaction de celle-ci avec un chlorure, iodure ou bromure de méthyl-magnésium, dans les conditions d'une réaction dite de Grignard. L'alcool tertiaire de formule

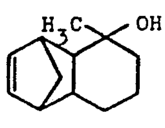

(Ia)

résultant de ladite addition est ensuite estérifié selon les techniques usuelles, par traitement au moyen d'un composé de formule

R—Y (II)

dans laquelle le symbole Y représente un atome d'halogène, le chlore ou le brome par exemple, ou un radical O-acétyle et R un radical acyle dérivant d'un acide carboxylique saturé ou insaturé contenant de 1 à 6 atomes de carbone. C'est ainsi qu'en traitant l'alcool tertiaire (Ia) ci-dessus au moyen d'anhydride mixte formique-acétique (Y = O-acétyle; R = formyle dans la formule (II)), on obtient le formiate de 3-méthyl-tricyclo[6.2.1.$0^{2,7}$]undéc-9-ène-3-yle. De façon analogue, le traitement dudit alcool (Ia) au moyen de chlorure d'acétyle (Y = Cl; R = acétyle dans la formule (II)) conduit à la préparation d'acétate de 3-méthyl-tricyclo[6.2.1.$0^{2,7}$]undéc-9-ène-3-yle. Il en est de même pour l'obtention du propionate, du butyrate, du valérate, du caproate, de, l'isobutyrate, de l'acrylate et du méthacrylate correspondants.

Pour l'obtention des esters saturés, on procède premièrement à la réduction de l'alcool (Ia) ci-dessus, par exemple par hydrogénation en présence d'un catalyseur tel le palladium sur charbon, le nickel de Raney ou l'oxyde de platine. L'alcool tertiaire résultant de formule

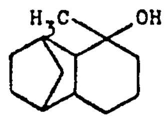

(Ib)

est finalement estérifié comme indiqué précédemment. Selon les cas, on peut également procéder directement à l'hydrogénation de l'ester insaturé correspondant.

La tricyclo[6.2.1.$0^{2,7}$]undéc-9-ène-3-one utilisée comme produit de départ est un composé connu et peut notamment se préparer à partir de cyclohex-2-ène-1-one et de cyclopentadiène selon une méthode décrite dans la littérature (voir à ce sujet J.Org.Chem. *39*, 3063 (1974) et DE—OS no. 27 37 525).

L'invention sera illustrée de façon plus détaillée à l'aide des exemples ci-après (températures en degrés centigrades).

### Exemple 1
### 3-Méthyl-tricyclo[6.2.1.$0^{2,7}$]undéc-9-ène-3-ol

A une suspension de 4,55 g (0,187 mole) de magnésium dans 10 ml d'éther absolu, on a ajouté goutte à goutte 28 g (0,197 mole) de iodure de méthyle en solution dans 50 ml d'éther absolu. Après dissolution de la totalité du magnésium on a ajouté 26 g (0,160 mole) de tricyclo[6.2.1.$0^{2,7}$]undéc-9-ène-3-one en solution dans 150 ml d'éther. L'addition s'est effectuée à reflux et sous bonne agitation. Après avoir maintenu le chauffage à reflux durant 24 h, le mélange réactionnel refroidi à température ambiante a été décomposé par addition à 200 ml d'une solution aqueuse à 20% de $NH_4Cl$, maintenue à 0°. Après séparation de la phase organique, lavage de l'extrait aqueux à l'éther (3 × 50 ml), lavage des extraits organiques réunis au moyen de $H_2O$ (3 × 50 ml), séchage sur $Na_2SO_4$ et évaporation des parties volatiles, on a recueilli 27 g de résidu.

Ce dernier a été purifié comme suit: les 27 g de résidu obtenu ci-dessus ont été premièrement mélangés à 27 g de réactif de Girard P, 27 ml d'acide acétique et 270 ml d'éthanol, puis chauffés sous reflux durant 24 h. Après évaporation à sec du mélange réactionnel, le résidu a été repris dans 50 ml de $H_2O$. On a procédé ensuite à une extraction à l'éther (4 × 50 ml), suivie d'un lavage avec $H_2O$ (3 × 50 ml), puis $NaHCO_3$ à 10% dans l'eau (3 × 50 ml) et encore $H_2O$. Après séchage sur $Na_2SO_4$, filtration et élimination des parties volatiles, la distillation du résidu a fourni 14,7 g du produit désiré (rdt. 51%). Eb. 55—60°/0,4 mbar (0,3 Torr).

IR: 3448, 2857, 1449, 1366, 1134, 1022, 816 $cm^{-1}$.

RMN: 1,2 (3H, s); 1,3—2,0 (8H, m); 2,0—3,0 (5H, m); 6,2 (2H, m) $\delta$ ppm.

SM: m/e = 97 (13), 95 (90), 94 (17), 79 (18), 67 (16), 66 (100), 43 (20).

### Exemple 2
### Formiate de 3-méthyl-tricyclo[6.2.1.$0^{2,7}$]undéc-9-ène-3-yle

On a premièrement ajouté 13 g (0,127 mole) d'anhydride acétique à 6 g (0,128 mole) d'acide formique à 98%, sous bonne agitation et en maintenant la température du mélange réactionnel à 0°, ledit mélange étant finalement mis à reposer durant 1 nuit à −5°. On y a ensuite incorporé rapidement 7,4 g (0,042 mole) de 3-méthyl-tricyclo[6.2.1.$0^{2,7}$]undéc-9-ène-3-ol — voir Exemple 1 —, poursuivant l'agitation durant 3 jours à température ambiante, après addition des réactifs. Le mélange de réaction a été finalement décomposé par addition à 100 ml d'une solution aqueuse saturée de $Na_2CO_3$. Après extraction à l'éther (3×50 ml), lavage avec $H_2O$ (3×50 ml), séchage sur $Na_2SO_4$ et filtration, on a isolé env. 8 g de produit brut. Après distillation sous pression réduite 0,027 mbar (0,02 Torr) on a recueilli le produit désiré avec un rendement de 58% env.

IR: 2910, 1714, 1440, 1236, 1170, 1115, 1020, 910, 895, 782 $cm^{-1}$.

RMN: 1,0—3,1 (12H, plusieurs m); 1,6 (3H, s); 6,12 (2H, m); 8,05 (1H, d) $\delta$ ppm

SM: m/e = 160 (11), 134 (9), 117 (8), 95 (100), 94 (71), 79 (53), 66 (86), 53 (7), 39 (13).

### Exemple 3
### Acétate de 3-méthyl-tricyclo[6.2.1.$0^{2,7}$]undéc-9-ène-3-yle

10 g (0,056 mole) du 3-méthyl-tricyclo[6.2.1.$0^{2,7}$]undéc-9-ène-3-ol obtenu à l'Exemple 1 ont été mélangés à 77 g (0,636 mole) de N,N-diméthyl-aniline, 77 g (0,980 mole) de chlorure d'acétyle et 400 ml de chloroforme. Après addition des réactifs, le mélange a été chauffé durant 4 h à la température de reflux. Après refroidissement à température ambiante, le mélange a été versé sur 300 ml d'eau glacée. La phase organique a été extraite au moyen de $CH_2Cl_2$ (3 × 100 ml), lavée avec HCl à 10% dans $H_2O$, neutralisée au moyen de $NaHCO_3$ à 10% dans l'eau et finalement $H_2O$ (3 × 50 ml). Après séchage sur $Na_2SO_4$, filtration et évaporation à sec, on a recueilli 11,7 g de produit brut. Par distillation fractionnée, on a finalement isolé 6,8 g (rdt. env. 55%) du produit désiré, Eb. 55—60° 0,027 mbar (0,02 Torr).

IR: 2915, 1724, 1570, 1449, 1362, 1239, 1117, 1022, 939, 897, 782 $cm^{-1}$.

RMN: 1,0—1,5 (5H, m); 1,5 (4H, s); 2,0 (4H, m); 2,1—3,0 (5H, m); 6,1 (2H, m) $\delta$ ppm

SM: m/e = 160 (17), 95 (100), 94 (88), 91 (17), 79 (64), 66 (82), 43 (46).

## Exemple 4

### Propionate de 3-méthyl-tricyclo[6.2.1.$0^{2,7}$]undéc-9-ène-3-yle

47 g (0,50 mole) de chlorure de propionate ont été ajoutés, à température ambiante, à un mélange de 5 g (0,028 mole) de 3-méthyl-tricyclo[6.2.1.$0^{2,7}$]undéc-9-ène-3-ol — voir Exemple 1 —, 39 g (0,32 mole) de N,N-diméthylaniline et 200 ml de chloroforme. Après avoir été chauffé durant 18 h à reflux, le mélange réactionnel a été amené à température ambiante et finalement versé sur 200 g de glace. Après extraction au moyen de $CH_2Cl_2$ (3×30 ml), lavage de la phase organique avec HCl à 10% dans l'eau (3 × 20 ml), $Na_2CO_3$ à 20% dans l'eau (100 ml), HCl à 10% dans l'eau (3 × 20 ml) et finalement $H_2O$ (5×30 ml), séchage et évaporation, on a recueilli 6,5 g de résidu brut.

Après distillation, Eb. 70—75°/0,13 mbar (0,1 Torr) suivie d'une chromatographie sur gel de silice (éluant cyclohexane/éther 7:3), on a finalement isolé 2,24 g (rdt. 34%) du produit désiré.

Eb. 65—68°/0,1 Torr; F env. 30°

IR: 2882, 1712, 1449, 1333, 1181, 1114 $cm^{-1}$.

RMN: 1,08 (3H, t, J=7Hz), 1,53 (3H, s); 1,1—1,6 et 1,8—3,0 (14H); 6,03 (2H, m) $\delta$ ppm

SM: m/e = 125 (17), 95 (34), 81 (16), 73 (21), 55 (31), 43 (100).

## Exemple 5

### Acrylate de 3-méthyl-tricyclo[6.2.1.$0^{2,7}$]undéc-9-ène-3-yle

46 g (0,50 mole) de chlorure d'acrylyle ont été ajoutés, à température ambiante, à 5 g (0,028 mole) de 3-méthyl-tricyclo[6.2.1.$0^{2,7}$]undéc-9-ène-3-ol — voir Exemple 1 — et 39 g (0,32 mole) de N,N-diméthyl-aniline en solution dans 200 ml de $CHCl_3$. Après chauffage à reflux durant 18 h, refroidissement et addition sur 200 g de glace, le mélange réactionnel a été traité comme indiqué à l'Exemple 4 pour donner finalement 7 g de résidu brut.

Après distillation, Eb. 65—75°/0,13 mbar (0,1 Torr) suivie d'une purification par chromatographie sur gel de silice (éluant cyclohexane/éther 7:3), on a finalement isolé 1,9 g (rdt. 29%) du produit désiré.

Eb. 68—70°/0,13 mbar (0,1 Torr); F 41—42°

IR: 2899, 1704, 1613, 1443, 1395, 1198 $cm^{-1}$.

RMN: 1,1—3,1 (12H), 1,61 (3H, s); 5,56—6,53 (5H) $\delta$ ppm

SM: m/e = 160 (12), 95 (100), 94 (72), 79 (49), 66 (72), 55 (42).

## Exemple 6

### Isobutyrate de 3-méthyl-tricyclo[6.2.1.$0^{2,7}$]undéc-9-ène-3-yle

A 10 g (0,056 mole) de 3-méthyl-tricyclo[6.2.1.$0^{2,7}$]undéc-9-ène-3-ol — voir Exemple 1 — en mélange avec 10 g (0,083 mole) de N,N-diméthyl-aniline et 50 ml de $CHCl_3$, on a rapidement ajouté 7,5 g (0,070 mole) de chlorure d'isobutyryle. Après avoir été chauffé à reflux durant 5 h, le mélange réactionnel a été évaporé à sec, puis repris dans 100 ml de $H_2SO_4$ à 10% dans l'eau. Après extraction à l'éther (3 × 100 ml), lavages successifs avec $H_2SO_4$ à 10% (5 × 30 ml), NaOH à 10% (4 × 30 ml) et finalement $H_2O$ (4 × 30 ml), séchage sur $Na_2SO_4$ et distillation, on a recueilli 8,0 g d'un produit ayant Eb. 60—85°/0,27 mbar (0,2 Torr).

Après purification par chromatographie sur gel de silice (éluant:cyclohexane/acétate d'éthyle 7:3) et évaporation, on a isolé 6,0 g (rdt. 43%) du produit désiré.

IR: 2899, 1724, 1460, 1339, 1248, 1136, 1062, 913 $cm^{-1}$

RMN: 1,0—1,2 (6H, 2s); 1,3—1,5 (6H, m); 1,6 (3H, s); 1,9—3,0 (7H, large m); 6,1 (2H, m) $\delta$ ppm

SM: m/e = 160 (11), 95 (100), 94 (48), 79 (26), 66 (59), 43 (42).

## Exemple 7

### 3-Méthyl-tricyclo[6.2.1.$0^{2,7}$]undécane-3-ol

25 g (0,140 mole) de 3-méthyl-tricyclo[6.2.1.$0^{2,7}$]undéc-9-ène-3-ol — voir Exemple 1 — en solution dans 150 ml de méthanol ont été hydrogénés à pression et température ambiantes, en présence de 2,5 g de palladium à 10% sur charbon. Après filtration du mélange réactionnel, évaporation et distillation, on a isolé 24 g d'une huile incolore Eb. 60—65°/0,13 mbar (0,1 Torr) contenant env. 90% du produit désiré selon l'analyse par chromatographie en phase gazeuse.

Un échantillon pour analyse a été purifié par distillation sur colonne à bande tournante.

IR: 3484, 2857, 1449, 1357, 1121, 926 $cm^{-1}$

RMN: 1,06 (1H, large s); 1,22 (3H, s); 1,2—2,5 (16H) $\delta$ ppm

SM: $M^+$ = 180 (23); m/e = 162 (63), 134 (72), 95 (97), 79 (60), 71 (100), 43 (98).

## Exemple 8

### Formiate de 3-méthyl-tricyclo[6.2.1.$0^{2,7}$]undéc-3-yle

8,7 g de formiate de 3-méthyl-tricyclo[6.2.1.$0^{2,7}$]undéc-9-ène-3-yle — produit brut; voir Exemple 2 — en solution dans 60 ml de méthanol, ont été hydrogénés à pression et température ambiantes en présence de 0,8 g de palladium à 10% sur charbon. Après filtration et évaporation, le résidu obtenu a été repris dans 100 ml d'éther et la phase organique lavée avec $NaHCO_3$ à 10% dans l'eau (20 ml), puis $H_2O$ (2×20 ml) et finalement séchée et évaporée pour donner 8 g de résidu brut.

7 g dudit résidu ont été purifiés par chromatographie sur gel de silice (éluant cyclohexane/éther 7:3) pour donner finalement 2,2 g (rdt. 25%) du produit désiré, Eb. 58—60°/0,067 mbar (0,05 Torr).
IR: 2857, 1704, 1449, 1370, 1183, 1111 $cm^{-1}$
RMN: 1,1—2,7 (16H); 1,57 (3H, s); 8,1 (1H, large s) $\delta$ ppm
SM: m/e = 162 (54), 134 (96), 119 (42), 95 (100), 79 (75), 67 (50), 41 (47).

Exemple 9

Acétate de 3-méthyl-tricyclo[6.2.1.$0^{2,7}$]undéc-3-yle

22 g (0,122 mole) de 3-méthyl-tricyclo[6.2.1.$0^{2,7}$]undécane-3-ol — voir Exemple 7 — et 220 ml d'acétate d'isopropényle ont été chauffés durant 6 h à reflux, en présence de 0,1 g d'acide p-toluène-sulfonique. Après refroidissement à température ambiante, le mélange réactionnel a été lavé au moyen de $NaHCO_3$ à 10% dans l'eau (30 ml), puis $H_2O$ (3×30 ml), séché sur $Na_2SO_4$ et évaporé pour donner 24 g de résidu brut.

Après distillation, on a recueilli 21 g d'un produit ayant Eb. 55—70°/0,067 mbar (0,05 Torr), finalement purifié par une nouvelle distillation en présence de $K_2CO_3$. On a ainsi isolé 13 g (rdt. 48%) du produit désiré.
IR: 2899, 1715, 1443, 1361, 1239, 1015 $cm^{-1}$
RMN: 1,1—2,8 (16H); 1,52 (3H, s); 1,98 (3H, s) $\delta$ ppm
SM: m/e = 162 (52), 134 (100), 119 (47), 106 (61), 95 (90), 79 (70), 43 (74).

Exemple 10

Les 3 composés ci-après ont été utilisés à titre d'ingrédients parfumants pour la préparation de savons de toilette.
Composé A: acétate de 3-méthyl-tricyclo[6.2.1.$0^{2,7}$]undéc-9-ène-3-yle
Composé B: acétate de 3-méthyl-tricyclo[6.2.1.$0^{2,7}$]undéc-3-yle
Composé C: formiate de 3-méthyl-tricyclo[6.2.1.$0^{2,7}$]undéc-9-ène-3-yle

Lesdits composés ont ensuite été utilisés comme suit (parties en poids):

| Ingrédients | Echant. A | Echant. B | Echant. C |
|---|---|---|---|
| Pâte à savon commerciale 1) | 100 | 100 | 100 |
| Composé A | 1 | – | – |
| Composé B | – | 1 | – |
| Composé C | – | – | 1 |

1) olfactivement neutre.

La pâte à savon ainsi parfumée a été façonnée en savons de toilette selon les techniques usuelles et ceux-ci finalement soumis à une évaluation olfactive. Les échantillons examinés ont été caractérisés comme indiqué ci-après:
Echant. A: odeur montante, fraîche et verte; rappelle par certains côtés l'essence de sauge sclarée.
Echant. B: odeur de type boisé, ambré, vert; rappelle l'essence de sauge sclarée
Echant. C: odeur de type boisé, ambré, avec note verte et aromatique; rappelle l'essence de marjolaine douce.

Exemple 11

On a préparé une poudre de détergent parfumée à partir des ingrédients mentionnés ci-après (parties en poids):

| Ingrédients | Echant. A | Echant. B | Echant. C |
|---|---|---|---|
| Poudre de détergent commerciale 1) | 100 | 100 | 100 |
| Composé A 2) | 0,15 | – | – |
| Composé B 2) | – | 0,15 | – |
| Composé C 2) | – | – | 0,15 |

1) contient enzymes et perborates.

2) voir Exemple 10.

Après mélange intime des ingrédients, les échantillons de poudre ainsi parfumée ont été soumis à une évaluation olfactive et finalement caractérisés comme suit:
Echant. A: odeur agréable, tout à la fois fraîche et verte, de type sauge sclarée
Echant. B: odeur légèrement boisée, de type vert, ambré et aromatique
Echant. C: odeur boisée et ambrée, avec note verte et aromatique, de type marjolaine douce.

On a pu constater en outre, dans les 3 cas ci-dessus, que l'effet olfactif perçu demeurait inchangé, après un stockage de plusieurs semaines dans les conditions usuelles d'emploi. Il a été également constaté que le parfum développé par les échantillons ci-dessus adhérait encore fort bien au linge, après lavage.

Exemple 12

On a procédé à la préparation de shampooing parfumé à partir des ingrédients ci-après (parties en poids):

| Ingrédients | Echant. A | Echant. B | Echant. C |
|---|---|---|---|
| Base pour shampooing commerciale 1) | 100 | 100 | 100 |
| Composé A 2) | 0,20 | – | – |
| Composé B 2) | – | 0,20 | – |
| Composé C 2) | – | – | 0,20 |

1) olfactivement neutre.

2) voir Exemple 10.

Les échantillons ainsi parfumés ont ensuite été soumis à une évaluation olfactive et finalement caractérisés comme suit:
Echant. A: odeur montante, fraîche et verte; type "shampooing aux herbes"
Echant. B: odeur légèrement boisée, verte et ambrée; type A
Echant. C: odeur boisée, ambrée, avec note verte et aromatique; type "médicinal".

Exemple 13

On a premièrement préparé un base parfumante pour Eau de Cologne en mélangeant les ingrédients ci-après:

| Ingrédients | Parties en poids |
|---|---|
| Essence de citron | 250 |
| Essence de bergamote | 300 |
| Essence d'orange | 150 |
| Petitgrain bigarade | 100 |
| Néroli bigarade | 20 |
| Essence de lavande | 70 |
| Essence de thym blanc | 10 |

En diluant la base ci-dessus à 3% dans l'alcool éthylique à 95%, on obtient une Eau de Cologne classique dont l'odeur peut être qualifiée de chaude et de type hespéridé.

En ajoutant 5 parties d'une solution d'acétate de 3-méthyl-tricyclo[6.2.1.$0^{2.7}$]undéc-9-ène-3-yle à 10% dans l'alcool éthylique à 95%, à 95 parties de la solution préparée ci-dessus, on obtient une Eau de Cologne dont l'odeur est devenue plus montante, acquérant un caractère de type masculin particulièrement plaisant.

Un effet olfactif identique a été obtenu en remplaçant dans l'exemple ci-dessus l'acétate de 3-méthyl-tricyclo[6.2.1.$0^{2.7}$]undéc-9-ène-3-yle par l'acétate de 3-méthyl-tricyclo[6.2.1.$0^{2.7}$]undéc-3-yle ou le formiate de 3-méthyl-tricyclo[6.2.1.$0^{2.7}$]undéc-9-ène-3-yle.

Exemple 14

Un composition parfumante de base pour Eau de toilette masculine a été préparée en mélangeant les ingrédients ci-après (parties en poids):

| | |
|---|---|
| Acétate de linalyle | 100 |
| Absolu de mousse de chêne à 50%* | 80 |
| Acétate de p-t-butyl-cyclohexyle | 70 |
| Essence de citron | 70 |
| Acétate de cédryle | 60 |
| Essence d'orange de Floride | 50 |
| Essence de lavande | 50 |
| Essence de bergamote synthétique | 50 |
| Acétate d'isobornyle | 40 |
| Exaltex®[1)] | 40 |
| Musc ambrette | 30 |
| Essence de santal oriental | 60 |
| Eugénol | 20 |
| Essence de thym blanc à 10%* | 20 |
| β-Damascénone à 1%* | 20 |
| Essence de néroli Portugal | 10 |
| Coumarine | 10 |
| Absolu de lavande | 10 |
| Ambrox®[1)] à 1%* | 10 |
| Total | 800 |

* dans le phtalate de diéthyle
[1)] origine: Firmenich SA, Genève — Suisse

La base ainsi préparée développe une odeur que l'on peut qualifier de type chypré. En ajoutant à 80 parties de ladite base 20 parties d'acétate de 3-méthyl-tricyclo[6.2.1.$0^{2,7}$]undéc-9-ène-3-yle, on obtient une nouvelle composition parfumante développant une odeur plus riche, plus harmonieuse et plus arrondie.

En remplaçant dans l'Exemple ci-dessus l'acétate de 3-méthyl-tricyclo[6.2.1.$0^{2,7}$]undéc-9-ène-3-yle par une quantité identique d'acétate de 3-méthyl-tricyclo[6.2.1.$0^{2,7}$]undéc-3-yle ou de formiate de 3-méthyl-tricyclo[6.2.1.$0^{2,7}$]undéc-9-ène-3-yle, on a observé un effet similaire, la composition obtenue s'étant enrichie d'une note de type boisé et ambré fort agréable.

**Revendications**

1. Composés de formule

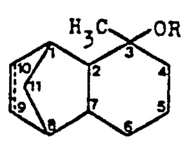

(I)

possédant une simple ou double liaison dans la position indiquée par les pointillés et dans laquelle le symbole R représente un atome d'hydrogène ou un radical acyle dérivant d'un acide carboxylique saturé ou insaturé contenant de 1 à 6 atomes de carbone.

2. Formiate de 3-méthyl-tricyclo[6.2.1.$0^{2,7}$]undéc-9-ène-3-yle.

3. Acétate de 3-méthyl-tricyclo[6.2.1.$0^{2,7}$]undéc-9-ène-3-yle.

4. Acétate de 3-méthyl-tricyclo[6.2.1.$0^{2,7}$]undéc-3-yle.

5. Utilisation d'un composé de formule (I), telle que définie à la revendication 1, à titre d'ingrédient parfumant, respectivement aromatisant, pour la préparation de parfums, de produits parfumés, d'arômes artificiels ou pour l'aromatisation d'aliments, de boissons, de préparations pharmaceutiques et du tabac.

6. Composition parfumante, respectivement aromatisante, caractérisée en ce qu'elle contient à titre d'ingrédient actif un composé de formule (I) telle que définie à la revendication 1.

**Patentansprüche**

1. Verbindungen der Formel (I)

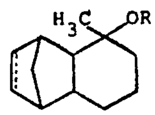

(I)

mit einer Einfach- oder einer Doppelbindung in der durch gestrichelte Linien dargestellten Stellung, worin R Wasserstoff oder eine Acyl Gruppe, die sich von einer gesättigten oder ungesättigten Carbonsäure mit 1 bis 6 Kohlenstoffatomen ableitet, bezeichnet.

2. 3-Methyl-tricyclo[6.2.1.$0^{2,7}$]undec-9-en-3-yl Formiat.

3. 3-Methyl-tricyclo[6.2.1.$0^{2,7}$]undec-9-en-3-yl Acetat.

4. 3-Methyl-tricyclo[6.2.1.$0^{2,7}$]undec-3-yl Acetat.

5. Verwendung einer Verbindung der Formel (I) gemäss Anspruch 1, als Riechstoff, bzw. Geschmacksstoff, für die Herstellung von Parfums, parfümierten Produkten, künstlichen Aromen oder für die Aromatisierung von Lebensmitteln, Getränken, pharmazeutischen Präparaten und Tabak.

6. Parfüm-, bzw. Aromakomposition, dadurch gekennzeichnet, dass sie als wirksamer Bestandteil eine Verbindung der Formel (I) gemäss Anspruch 1 enthält.

**Claims**

1. Compounds of formula (I)

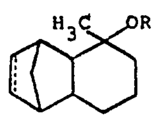

(I)

having a single or a double bond in the position indicated by the dotted line and wherein symbol R represents a hydrogen atom or an acyl radical derived from a saturated or an unsaturated carboxylic acid having 1 to 6 carbon atoms.

2. 3-Methyl-tricyclo[6.2.1.$0^{2,7}$]undec-9-en-3-yl formate.

3. 3-Methyl-tricyclo[6.2.1.$0^{2,7}$]undec-9-en-3-yl acetate.

4. 3-Methyl-tricyclo[6.2.1.$0^{2,7}$]undec-3-yl acetate.

5. Utilization of a compound of formula (I) according to claim 1 as a perfuming or a flavouring ingredient for the preparation of perfumes, perfumed products, artificial flavours or for the aromatization of foodstuffs, beverages, pharmaceutical preparations and tobacco.

6. Perfuming or flavouring composition characterized in that it contains as active ingredient a compound of formula (I) as defined in claim 1.